# EUROPEAN PATENT SPECIFICATION

(11) **EP 3 086 659 B1**
(45) Date of publication and mention of the grant of the patent: **17.07.2019**
(21) Application number: 14833259.6
(22) Date of filing: 19.12.2014
(51) Int. Cl.: A61K 31/12, A61K 31/198, A61K 31/205, A61K 31/4525, A61K 31/525, A61K 36/67, A61K 36/9066, A61K 38/01, A61K 38/38, A61K 45/06, A23L 5/00, A61K 9/16, A61P 11/00, A61P 39/06, A61P 29/00, A61K 38/17, A61K 9/00, A61K 35/20, A23L 33/10, A23L 33/185

(54) **NUTRITIONAL COMPOSITION FOR THE PREVENTION AND TREATMENT OF COPD AND RELATED SYMPTOMS**
ERNÄHRUNGSZUSAMMENSETZUNG ZUR VORBEUGUNG UND BEHANDLUNG VON COPD UND ASSOZIIERTEN SYMPTOMEN
COMPOSITION NUTRITIONNELLE POUR LA PRÉVENTION ET LE TRAITEMENT DE LA BPCO ET DES SYMPTÔMES ASSOCIÉS

(30) Priority: 20.12.2013 IT MI20132169
(43) Date of publication of application: 02.11.2016
(73) Proprietor: Thompson S.r.L., 00196 Roma (IT)
(72) Inventor: SORTINO, Enrico, I-95128 Catania (IT); SCAPAGNINI, Giovanni, I-95131 Catania (IT)
(74) Representative: Coppo, Alessandro
(86) International application number: PCT/IB2014/067148
(87) International publication number: WO 2015/092763

(56) References cited:
- US-A1- 2003 003 166
- RAHMAN I ET AL: "ANTIOXIDANT THERAPEUTIC TARGETS IN COPD", CURRENT DRUG TARGETS, BENTHAM SCIENCE PUBLISHER, US, vol. 7, no. 6, 1 June 2006 (2006-06-01), pages 707-720, XP009069239, ISSN: 1389-4501, DOI: 10.2174/138945006777435254
- KHERADPEZHOUH E ET AL: "Curcumin protects rats against acetaminophen-induced hepatorenal damages and shows synergistic activity with N-acetyl cysteine", EUROPEAN JOURNAL OF PHARMACOLOGY, ELSEVIER SCIENCE, NL, vol. 628, no. 1-3, 25 February 2010 (2010-02-25), pages 274-281, XP026851677, ISSN: 0014-2999 [retrieved on 2009-11-15]
- LOUIS LAVIOLETTE ET AL: "Combined Effect of Dietary Supplementation with Pressurized Whey and Exercise Training in Chronic Obstructive Pulmonary Disease: A Randomized, Controlled, Double-Blind Pilot Study", JOURNAL OF MEDICINAL FOOD, vol. 13, no. 3, 1 June 2010 (2010-06-01), pages 589-598, XP055179659, ISSN: 1096-620X, DOI: 10.1089/jmf.2009.0142
- SUGAWARA KEIYU ET AL: "Effect of anti-inflammatory supplementation with whey peptide and exercise therapy in patients with COPD", RESPIRATORY MEDICINE, vol. 106, no. 11, 3 August 2012 (2012-08-03), pages 1526-1534, XP028937949, ISSN: 0954-6111, DOI: 10.1016/J.RMED.2012.07.001

## Description

### FIELD OF THE INVENTION

The present invention relates to a nutritional composition for preventing or treating chronic obstructive pulmonary disease and related symptoms.

The present invention originates in the field of nutritional products, in particular for medical use.

In particular, the present invention relates to a food for special medical purposes useful in the prevention or treatment of chronic obstructive pulmonary disease and symptoms associated with this disease.

### PRIOR ART

Chronic obstructive pulmonary disease, referred to below as COPD, is a pathological condition characterised by chronic airflow obstruction that is usually progressive and associated with an increased inflammatory response in the airways and lung to particular gas or noxae.

COPD is a condition whose incidence is increasing, in 1990 it was the sixth leading cause of death worldwide and WHO projections indicate that in 2020 it will become the third leading cause of death, after cancer and major cardiovascular diseases.

Among the risk factors, the main one is represented by the exposure of active and passive, even previous, to tobacco smoke. The significant individual response to various etiological noxae has nevertheless stimulated the search for other risk factors. These were divided into environmental and individual ones. Among the former, occupational exposure to chemicals, organic and inorganic dusts, in addition to environmental or domestic pollution are important. Among the latter, the alpha-1 antitrypsin deficiency, low birth weight, respiratory infections in early childhood, nutritional and socio economic status, familiarity and sex are of decreasing relevance.

The symptomatology that makes one suspect the existence of a COPD is represented by chronic, mainly productive cough, especially in the forms in which the bronchitis component prevails; dyspnoea worsening over time; paroxysmal dyspnoea and persistent cough with little viscous and mucus sputum production, especially in the forms in which chronic bronchial asthma prevails. Although symptoms are often present, their absence does not exclude the diagnosis of COPD. This is given by spirometry, after administration of a bronchodilator, in a ratio of FEV1/FVC < 0.70.

Once diagnosed, the disease is divided into functional stages, which is of great importance from the prognosis and therapeutic point of view. According to the GOLD 2013 guidelines, there are four distinct stages:
- Stage I (mild COPD) in which the post-bronchodilator FEV1 is equal to or greater than 80% of the theoretical value;
- Stage II (moderate COPD) in which the post-bronchodilator FEV1 is in the range between 50 and 70% of the theoretical value;
- Stage III (severe COPD) in which the post-bronchodilator FEV1 is in the range between 30 and 49% of the theoretical value;
- Stage IV (very severe COPD) in which the post-bronchodilator FEV1 is < 30% of the theoretical value or in which a post-bronchodilator FEV1 between 30 and 50% of the theoretical value is associated with respiratory failure (PaO < 60 mm Hg).

In all four stages of the disease, the existence of a growing stress and oxidative damage of the body was then verified which occurs in the lungs causing bronchial constriction, stimulating the conversion of arachidonic acid into isoprostanes. Oxidative stress also causes a parenchymal destruction and pro-inflammatory action.

The prognosis of COPD is related to the severity of symptoms and spirometric alterations, the risk of exacerbations and the presence of co-morbidity and systemic effects.

From a pathophysiological point of view, the alterations found in COPD are a consequence of both inflammatory alterations and emphysematous ones.

Inflammatory phenomena cause, at a bronchial level, a dysfunction of the mucociliary apparatus. The destruction of the walls of the alveoli and of the alveolar attachments supporting the respiratory bronchioles is the pathophysiological basis of the decrease in the expiratory airflow which is the most characteristic alteration of COPD.

This results in a marked increase in the total resistance of the small airways, and a consequent increase in the work of the respiratory muscles, accompanied by a reduction in their coordination and a decrease in the FEV/FVC ratio. This framework also causes a decrease in lung capacity and a pulmonary inability to perform gas exchange, leading to hypoxia and vasoconstriction and related illnesses.

Furthermore, in the forms of COPD predominantly of the emphysematous type there is a muscle hypotrophy, as a consequence of chronic inflammation.

Given this complex symptomatology and stage of PBCO, different drug treatments are employed, aimed at reducing symptoms and preventing possible exacerbations of the disease.

Currently, the most commonly used pharmacological treatments provide for the administration of bronchodilators, such as theophylline or beta-2 antagonists with long or short duration of action as a function of the need to control the respiratory symptoms of the disease or treat respiratory difficulties.

Inhaled or oral corticosteroids are also administered in cases of exacerbation of the symptoms of COPD.

Vaccines, in particular anti-influenza and pneumococcal ones, are also used to limit the respiratory risks associated with airway infections and antibiotics if necessary, particularly in case of disease flare-up in the presence of infection. The use of mucolytics is also provided in order to make bronchial secretions viscous and clear the airways of mucus and phlegm.

The conventional drug therapies currently used to treat COPD are not, however, devoid of drawbacks.

For example it was observed that the use of bronchodilators in minor cases can promote an excessive dilation of bronchi and bronchioles, leaving the passage of pollutants present in the air environment to lung areas pervious.

Furthermore, the systemic corticosteroid therapy can lead to the development of both acute myopathy with rhabdomyolysis and chronic, in addition to a weakening of the immune response.

Conversely, repeated courses of antibiotics can lead to the formation of pathogenic strains resistant to common antibiotics and lead to the formation of superinfection, with not infrequent adverse outcomes.

Mucolytic therapies commonly practiced, while leading to immediate relief in certain pathological conditions, do not produce any real improvement in the overall condition of the patient since they have virtually no effect on the complex symptoms of COPD not closely related to respiratory phenomena.

Furthermore, most of the available pharmacological treatments are directed to the treatment of stages II to IV with medium-high severity while there is a substantial absence of appropriate remedies for treating stage I of lesser severity.

Currently, there is the need for remedies or preparations suitable for preventing and treating COPD, particularly in the early stage, and related symptoms.

RAHMAN I ET AL: "Antioxidant Therapeutic Targets in COPD", CURRENT DRUG TARGETS, Bentham Science Publisher, US, vol. 7, no. 6, 1 June 2006 (2006-06-01), pages 707-720, discloses the use of antioxidants for the treatment of COPD.

It is a general object of the present invention to provide a composition suitable for improving the clinical condition and/or the symptoms associated with COPD. Another object of the present invention is to provide a composition suitable for treating or preventing COPD, particularly in stage I, based on active ingredients almost free of side effects on the human body and which allows the long-term treatment of the disease.

Is a further object of the present invention to provide a composition for use in a method of treatment for preventing or treating COPD and/or the symptoms associated therewith.

### SUMMARY OF THE INVENTION

According to certain aspects of the present invention, the inventors found that by providing a combination of N-acetylcysteine (NAC) and curcumin some defence mechanisms against oxidative stress are activated in the human body, giving a reduction of inflammation and protein hypercatabolism, which find specific use in the treatment and prevention of chronic obstructive pulmonary disease (COPD) and symptoms or disorders associated with this disease.

According to a first aspect, the present invention therefore provides a composition or nutritional product as defined in claim 1.

The combination of the antioxidant and protein hypercatabolism-inhibiting component a) with the protein-based component b) and vitamin B2 and carnitine and/or L-carnitine, restores a physiological protein metabolism, reduces alterations of muscle mass leading to respiratory failure of the individual suffering from COPD.

According to certain aspects of the invention, the inventors found that the administration of the Nac/Nal, curcumin mixture with a specific protein component and vitamin B2 and carnitine and/or L-carnitine, a considerable improvement of the general conditions of individuals affected by chronic pulmonary disease and their quality of life is achieved.

The composition of the invention combines the inhibitory effect on protein catabolism of component a) with the protein supply of component b), and vitamin B2 and carnitine and/or L-carnitine, giving a significant slowdown in the muscle hypotrophy and myopathy which, if untreated, lead to the reduction of the ventilatory capacity and dyspnoea in the patient suffering from COPD.

According to some aspects, the administration of the composition of the invention in COPD patients with respiratory failure facilitates the weaning of mechanical ventilation, improving muscle strength and exercise tolerance.

Furthermore, the anti-inflammatory and antioxidative action of N-acetylcysteine or a physiologically acceptable salt thereof, and curcumin is further enhanced by piperine since the latter increases the bioavailability of curcumin, determining a synergistic cell antioxidant effect.

According to some embodiments, the composition of the invention comprises N-acetylcysteine optionally in the form of a lysine salt, curcumin and piperine as synergist agent of the anti-inflammatory and antioxidative activity, in addition to a protein-based component and vitamin B2 and carnitine and/or L-carnitine.

In certain embodiments, the composition of the invention is a nutritional product or a dietary or food supplement which includes a component with antioxidant activity combined with a protein based nutritional component.

The proteins present in the nutritional composition are proteins derived from whey.

The composition of the invention further comprises riboflavin.

Typically, in the composition of the invention the active ingredients with synergistic activity are provided in a pharmaceutically, dietetically or nutritionally effective quantity.

The composition of the invention finds application in the prevention or treatment of COPD or as an adjuvant for the symptoms associated with this disease. According to some embodiments, the composition of the invention is a pharmaceutical composition, a nutritional product, a dietary supplement or a food for special purposes, that can be introduced into the diet of an individual suffering from COPD to improve the symptoms related to this pathology.

According to some aspects of the invention, there is provided a dietary preparation or kit containing N-acetylcysteine and/or its lysine salt, curcumin and a Protein-based component b) as defined in the claims, and vitamin B2 and carnitine and/or L-carnitine as a combined preparation for simultaneous, separate or subsequent use in the prophylaxis or treatment of COPD.

The present invention will be described in detail below with reference to the accompanying figures.

### BRIEF DESCRIPTION OF THE DRAWINGS

Some features and advantages of the present invention will become apparent from the accompanying drawings, in which:
Figure 1 shows histograms which illustrate the synergistic effect of a composition containing a combination of curcumin (CUR) and N-acetylcysteine (NAC) on the levels of intracellular glutathione in lung epithelial cells;
Figure 2 shows bar graphs which illustrate the increase in the time of expression of Nrf2 protein in nuclear extracts, curcumin and NAC taken individually and in combination, according to some aspects of the invention;
Figure 3 shows histograms which illustrate the increase in the expression of mRNA of Nrf2, HO-1 and GRX (d, e, f) and the comparison with the gene GAPDH;
Figure 4 shows a graphical representation of the pharmacological profile after oral administration of 2 g of curcumin (◆) and 2 g of curcumin with 20 mg of piperine (■) in healthy volunteers (n = 8). * P<0.01; **P<0.001;

### DETAILED DESCRIPTION OF THE INVENTION

According to some aspects of the invention, the inventors found that by administering N-acetylcysteine (NAC), optionally in the form of a salt of lysine (NAL), with curcumin, a synergistic effect is obtained on specific cellular mechanisms involved in inflammatory processes and in the cellular oxidative stress and on the protein metabolism of the human body which are typical of COPD and which are at the origin of its symptoms.

According to some aspects, the invention relates to a nutritional composition or dietary supplement comprising
a) a component with antioxidant and protein catabolism inhibiting activity comprising a synergistic combination of N-acetylcysteine or a salt thereof and curcumin,
b) a protein-based component containing proteins obtained from whey which prevents or treats the muscle hypertrophy, and vitamin B2 and carnitine and/or L-carnitine.

According to some aspects, the invention relates to the use of the composition described above in the treatment, prevention of COPD or weaning of mechanical ventilation or increase of the muscle mass in a patient suffering from COPD.

The inventors have also found that the combination of piperine with optionally salified N-acetylcysteine and curcumin leads to an increase in the bioavailability of curcumin giving a synergistic effect on cellular antioxidant and inhibition action of protein catabolism which has specific indication of use in the treatment, prevention of COPD.

A bioactive principle or component of the composition of the invention is N-acetylcysteine and/or a salt thereof, in particular lysine.

N-acetylcysteine (NAC) or acetylcysteine is an N-acetylated derivative of the cysteine amino acid which has a dual indirect mucolytic and antioxidant activity. The mucolytic action is carried out through the activity of its free sulfhydryl group, which exerts an activity of reduction of the disulphide bonds of mucoproteins, causing a split of glycoprotein molecules into smaller units. Through this mechanism of action, the rheological qualities of the mucus are modified, so that it becomes more fluid and easily expelled.

The antioxidant action of NAC is determined by the generation, by means of a liver deacetylase, of cysteine, a component, together with glycine and glutamic acid, of glutathione, and together with steroids and hydroperoxide lipids, of glutathione peroxidase, a key molecule in the antioxidant mechanisms of the body. The decrease in the oxidative biomarkers consequent to the intake of NAC/NAL is accompanied by a reduction in bronchial hypersecretion accompanied by an improvement of symptoms and of the indices of pulmonary function, as well as a decrease in the frequency of exacerbations.

N-acylsteine (NAL), the lysine salt of acetylcysteine, acts in a similar manner. Unlike NAC, NAL has a neutral pH in solution. NAL also has an anti-inflammatory activity, partly due to its inhibitory activity on the release of interleukin (IL) -8, oxidant-mediated in alveolar epithelial cells A549.

Typically, NAC has a capacity to regulate and restore the GSH levels which is independent of the Nrf2 pathway.

Typically, N-acetylcysteine (NAL) and/or the lysine salt of acetylcysteine (NAC) can both be present in the composition of the invention, or only one of them.

In certain embodiments, the NAC is obtained synthetically or chemically.

In some embodiments, the composition of the invention contains N-acetylcysteine and/or N-acylsteine, in an amount ranging from 10 to 3000 mg, from 100 to 1200, from 400 to 800 mg.

In some embodiments, the composition of the invention contains N-acetylcysteine and/or N-acylsteine, in an amount ranging from 0.001 to 30% by weight, from 0.01 to 15% by weight or from 0.1 to 10% by weight.

The composition of the invention contains curcumin, in particular as a synergistic agent of acetylcysteine or its lysine salt.

Curcumin or turmeric (1E, 6E)-1,7-bis- (4-hydroxy-3-methoxyphenyl)-epta-1,6-dien-3,5-dione is a substance that can be obtained by extraction for example from the plant turmeric (Curcuma domestica Valeton).

According to some embodiments, the composition of the invention comprises curcumin, for example contained in a plant extract. In other embodiments, curcumin is synthetically or chemically produced.

A suitable plant extract containing curcumin can be obtained by extraction from a part of the Curcuma Longa plant, in particular from its root stock.

In some embodiments, the plant extract is a dry extract, for example titrated with 95% curcumin, obtained by grinding and drying a portion of the plant, in particular the root stock.

In some embodiments, the extraction of curcumin from a portion of the Curcuma plant can be carried out by conventional techniques, for example by extraction with a suitable solvent from portions of the plant, for example as indicated by Bohlman and Zdero in Phytochemestry 1982; 21: 2543-49.

In some embodiments, the composition of the invention contains a dry extract of Curcuma Longa, titrated to 95% of curcumin in an amount ranging from 10 to 3000 mg, from 100 to 1200, from 300 to 800 mg.

In some embodiments, the composition of the invention contains curcumin, in an amount ranging from 0.001 to 30% by weight, from 0.01 to 15% by weight or from 0.1 to 10% by weight.

Curcumin within the composition of the invention has an antioxidant action both direct, through the inhibition of the synthesis of eicosanoids and lipid peroxidation, and indirect through the induction of nuclear translocation of the Nuclear Factor Erithroid Derived - like 2 or Nfr2, an enzyme that controls more than 50 genes in lung tissue, with a consequent increase in the synthesis of glutathione, a molecule with high antioxidant activity. This activity involves the acceptance of electrons from free radicals, which therefore undergo a process of reduction, by the reduced glutathione, which at the same time oxidises. Then, through the action of the glutathione reductase (GSR) enzyme, also subject to the Nfr2 regulating activity, the oxidised Glutathione in turn undergo a process of reduction through the acquisition of an electron by the NADPH, which at the same time oxidises, becoming NADP. This continuing capacity for autoregeneration helps to determine the biological importance of glutathione as an antioxidant.

Glutathione also allows the activity of the glutathione peroxidase, a selenium-containing enzyme, an element able to neutralise the hydrogen peroxide with release of water, and other organic peroxides, free or bound to phospholipids of membranes. During the process of neutralisation of the peroxide, oxidised Glutathione is formed, which subsequently, under the action of Glutathione reductase, regenerates the reduced Glutathione.

The inventors have found that by associating or combining curcumin with NAC or NAL, a synergistic effect is obtained on the synthesis of glutathione, mediated by the expression of some defensive genes, enabled by the translocation from the cytoplasm to the nucleus of Nfr2. The nuclear translocation of Nfr2 by inducer genes results in an increase in the synthesis of Glutathione and in the activation of an important endogenous mechanism of defence against oxidative stress.

This mechanism is of particular importance in the treatment of COPD because the lack of activation of the antioxidant defences is an important pathogenesis element of the disease.

Moreover curcumin, by suppressing the activation of the transcription factor of the nuclear kappa factor B (NFkB), determines the inhibition of cyclooxygenase 2 (COX-2) and of other interleukins, resulting in a considerable anti-inflammatory effect that is added to the antioxidant effect, helping to reduce the symptoms and some of the underlying causes of COPD.

Moreover, curcumin prevents weight loss and muscle hypotrophy as it reduces or inhibits the protein hypercatabolism, typical of patients with COPD, acting at the level of the proteasome system - ubiquitin, typically through the signal pathway PI3K/AKT.

In some embodiments, curcumin is present or is supplied in the form of plant extract of the plant Turmeric.

The inventors have found that the combination of NAC or NAL and curcumin or a plant extract from Curcuma Longa acts synergistically to inhibit the activation of NF-kB and the expression of pro-inflammatory cytokines, and induces the translocation of Nrf2 and its antioxidant and anti-inflammatory genes in a cellular model of inflammation, surprisingly resulting in a regression of the main one related to COPD. This effect is particularly evident for the forms of COPD in stage I.

The Applicant has found a synergistic activity of NAC and/or NAL and curcumin on the prevention and/or treatment of symptoms related to COPD which may be found on specific biological parameters measured both at a cellular level and at a clinical level.

In the present application, the term "synergism or synergistic activity" means an activity that is greater than the sum of the activities of the individual active ingredient. In particular, the synergism occurs when at least two substances or active ingredients interact in a way that enhances or increases one or more of their effects. Therefore, two substances or active ingredients which produce apparently similar effects sometimes will produce exaggerated or reduced effects when used concurrently and a quantitative assessment is needed to distinguish these cases from a simple additive action (Tallarida RJ. Drug Synergism: its detection and applications. J PharmacolExpTher. 2001 Sep:298(3):865-72).

The inventors have also found that the synergistic effect of NAC or NAL and curcumin is further increased by matching or combining with piperine.

Piperine in fact, when given in combination with curcumin, increases the bioavailability thereof.

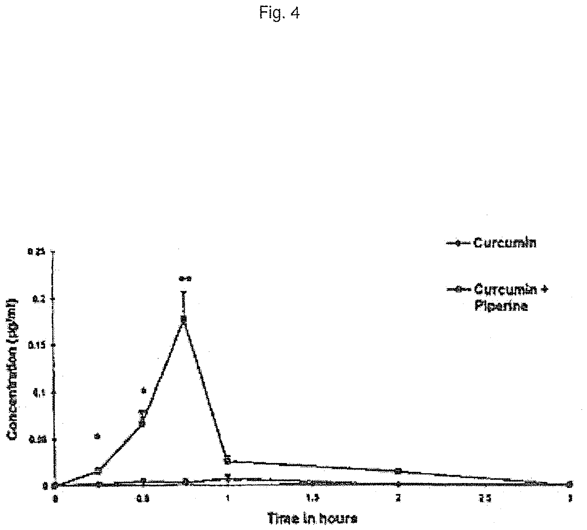

This synergistic effect is highlighted in figure 4, which shows in synthesis the results of a clinical study on volunteers on which the blood concentration of curcumin was tested after oral administration of 2 g of curcumin alone and 2 g of curcumin in combination with 20 mg of piperine. Serum levels of curcumin when administered orally alone were very low or undetectable in most withdrawals of the greater number of subjects, this explains the nearly flat serum concentration curve. However, when associated with piperine, the curcumin serum concentrations were significantly increased in the measurements up to 0.75 h; P <0.01 at 0.25 h and 0.5 h; P <0.001 at 0.75 h.

Subsequently, up to 1 h, a rapid decrease is observed followed by a gradual decline to 0 in the next three hours. The following Table lists the pharmacokinetic parameters (mean ± SEM).

**Table**

| Parameters | Curcumin | Curcumin (2 g/kg) + |
|---|---|---|
| Pharmacokinetic | (2g/kg) | Piperine (20 mg/kg) |
| Cmax (µg/ml) | 0.006±0.005 | 0.18±0.03 |
| Tmax (h) | 1 | 0.69±0.07 |
| t1/2(a) (h) | - | 0.11±0.02 |
| t1/2(el) (h) | - | 0.41±0.17 |
| | | |
| AUC(0-∞) (µg/h/ml) | 0.004 | 0.08±0.01 |
| Vd (L/kg) | - | 202.60±78.94 |
| Cl (L/h) | - | 7.33±1.25 |
| F (Relative Bioavailability) | | 2000% |

The table shows the pharmacokinetic parameters of curcumin when administered alone or with piperine. The maximum serum concentration, when administered alone, was just 0.006 µg/ml 1 hour after administration, while in combination with piperine, the maximum serum concentration (Cmax) had increased to 0.18 µg/ml and was reached earlier at 0.75 h from the administration. The volume of distribution (Vd) and the total clearance value (CI) were not calculated for curcumin administered alone as it was not possible to determine the serum levels in most of the measurements of the greater number of subjects. The area under the curve (AUC), however, was calculated using the trapezoidal method and had a value of 0.004 µg/h/ml and the relative bioavailability of curcumin when administered with the results shown in the diagram in fig. 4 and in the preceding table reflect an increase in the bioavailability of curcumin equal to 2000% in the subjects treated with the combination of curcumin and piperine.

Piperine (E, E)-1-[5-(1.3-benzodioxol-5-yl)-1-oxo-2 .4-pentadienyl]piperidine is an alkaloid of pepper, typically obtained from the black pepper plant.

According to some embodiments, the composition of the invention comprises piperine contained in a plant extract of black pepper, a plant in the family Piperaceae.

A suitable plant extract containing piperine can be obtained by extraction from a part of the black pepper plant, in particular from its fruit and/or seed.

In some embodiments, the plant extract is a dry extract, for example titrated with 95% piperine, obtained by grinding and drying a portion of the plant, in particular the fruit and/or seed.

In some embodiments, the composition of the invention contains a dry extract of black pepper, titrated to 95% of piperine in an amount ranging from 0.1 to 100 mg, from 0.5 to 50, from 1 to 10 mg.

In some embodiments, the composition of the invention contains piperine, in an amount ranging from 0.001 to 10% by weight, from 0.01 to 5% by weight or from 0.1 to 1% by weight.

In some embodiments, the extraction of curcumin from a portion of black pepper plant can be performed by conventional techniques, for example by extraction with a suitable solvent from portions of the plant, for example as indicated by Bohlman and Zdero in Phytochemestry 1982; 21: 2543-49.

Suitable solvents are organic, such as ethyl alcohol or methanol, dichloromethane, ethylacetate or dimethyl ketone, and mixtures thereof.

In particular, it was observed that piperine synergises the inhibition activity of curcumin of the prorinflammatory process of Nf-kb and also stimulates the activation of the Nrf2 anti-inflammatory process.

Accordingly, its incorporation in the formulation of the composition of the invention containing NAC or NAL and curcumin helps to increase the antioxidant and anti-inflammatory activity and thereby increases the effectiveness in preventing and/or treating COPD and related symptoms.

In some embodiments, the curcumin and piperine present in the composition of the invention are provided in the form of dried plant extracts.

Component b) of the composition of the invention is based on proteins. The protein component of the composition of the invention is provided to enhance or restore the physiological conditions of the skeletal muscles of the subject suffering from COPD, to regain muscle mass, to reduce the risk or delay the respiratory failure.

The proteins are whey proteins.

Suitable whey proteins are selected from the group consisting of Beta-lactoglobulins, Alpha-lactoglobulins, Lactoferrin, Glycomacropeptide, Immunoglobulins of classes IgG, IgA or IgM and mixtures thereof.

Whey proteins represent a source of oligopeptides fast absorbable by the human body because they are transported by high affinity carriers present in the gastrointestinal tract. In addition, whey protein contains all essential amino acids (EAA), have a high content of branched chain amino acids (BCAA), especially valine, leucine, isoleucine, which play a role in the protein synthesis activity in the muscle and contain a high amount of glutamine, an amino acid which intervenes in the synthesis of glutathione. They also provide the substrate necessary for protein synthesis and for gluconeogenesis and stimulate the synthesis of nitric acid.

In preferred embodiments, the proteins are selected from β-lactoglobulin, α-lactalbumin, serum albumin and mixtures thereof.

In some embodiments, the composition of the invention contains proteins obtained from whey, in an amount from 0.5 to 100 g, from 1 to 50, from 5 to 15 g.

In certain embodiments, proteins obtained from whey are present in a quantity from 1 to 95% by weight, from 5 to 80%, from 20 to 70% by weight of the composition.

In some embodiments, proteins obtained from whey are obtained by microfiltration of a fraction of whole milk.

The administration of proteins, obtained from whey, in particular together with other components of the composition of the invention, gives an increase of glutathione in the human body, a protein intake and an increase in insulin secretion, and a reduction of symptoms of COPD associated with the protein hypercatabolism, weight loss and muscle dysfunction.

The composition of the invention further comprises carnitine or (3R)-3-hydroxy-4-trimethylaminobutanoate, a short-chain carboxylic acid which acts as a carrier of fatty acids, allowing the mitochondria to use them for the production of ATP.

In particular, the main function of carnitine is to act as a carrier in the transport of NEFA from the cytoplasm to mitochondria, therefore allowing the Beta oxidation and the subsequent energy production. Consequently, carnitine plays an important energizing role useful for treating muscle disorders associated with COPD.

In some embodiments, the composition of the invention comprises further substances or active principles.

According to some embodiments, the composition of the invention further includes rosemary (*Rosmarinus officinalis),* or a plant extract from rosemary, especially from leaves.

In some embodiments, the composition of the invention comprises further active principles or substances, such as Co-Q10.

According to some embodiments, the composition of the invention further comprises one or more micronutrients and/or minerals such as salts of Mg, K, Na, Zn, Fe, Cr, Se, Mn and others.

In some embodiments, the composition of the invention comprises a physiologically acceptable and/or edible carrier.

The term "carrier" as used herein indicates a vehicle, excipient, diluent with which the components of the composition are administered.

Any carrier and/or excipient suitable for the desired preparation for administration to humans is contemplated for use with the compounds described in the present invention.

For purposes of the present application, the term "physiologically acceptable" is meant to indicate edible substances which are approved by health authorities for use in the pharmaceutical, nutritional or food applications.

Within the terms of the present application, the term combination or association means that one or more components or active ingredients are added to or mixed with one or other ingredients. The term association should not therefore be construed in the meaning that the active ingredients are associated with each other with the formation of bonds of chemical type or other type.

A physiologically acceptable carrier can be a pharmaceutically acceptable carrier.

The compositions of the present invention comprise any composition obtained by administering the combination of active principles of the present invention and a physiologically or pharmaceutically acceptable carrier. Such compositions are suitable for food, nutritional, pharmaceutical or dietetic use, in mammals, particularly in humans.

According to some embodiments, the composition of the invention is a food for special medical purposes.

The composition of the invention can take a variety of forms of preparation, depending on the desired route of administration.

For example for oral administration, the composition may be in solid form such as tablet, capsule, powder, granular, formulations with an extended release of the active ingredients. The compositions in solid form, in particular in granular or powder form, are preferred over other types of preparations.

The preparations in solid form may include one or more carriers such as starches, sugars, microcrystalline cellulose, and optionally diluents, granulating agents, lubricants, binders, disintegrating agents.

Tablets, pills, capsules, granules may also contain a binder such as acacia, tragacanth, cornstarch or gelatine; excipients such as calcium phosphate; a disintegrating agent such as cornstarch, potato starch, alginic acid; a lubricant such as magnesium stearate; a sweetening agent such as sucrose, lactose or saccharin. If desired, the tablets may be coated using traditional techniques.

When the pharmaceutical unit form is a capsule, it may contain a liquid carrier such as a fatty oil in addition to the above materials.

In the case of liquid preparations for oral administration, such as in the case of suspensions, emulsions, solutions, a suitable carrier may be selected from water, glycols, alcohol, oils and mixtures thereof.

In some embodiments, the composition of the invention is in liquid form, ready for administration.

Also flavouring agents, preservatives, colouring agents and the like may be present in the composition.

In some embodiments, plant extracts or active ingredients contained in the composition of the present invention can be combined or mixed as active ingredients, in intimate admixture with a suitable edible carrier and/or an excipient, according to pharmaceutical techniques and those used in the food or nutritional, traditional industry.

The compositions for pharmaceutical or nutritional use can be properly presented in single dosage form and prepared by any of the methods well known in the pharmaceutical or food field.

In some embodiments, the compositions or preparations of the invention can contain at least 0.1% of each active/bioactive substance. In these compositions the percentage of active ingredient may obviously vary for example from about 1% to about 60% by weight of the unit. The amount of active compound in such compositions is such that an effective dosage, as to prophylactic or therapeutic aspects, is obtained.

In some embodiments, the composition of the invention further comprises one or more additional components such as additives, fillers, stabilizers, emulsifiers, textured, film formers, plasticizers, wetting agents and thickeners.

Various other materials may be present as coatings or to modify the physical form of the pharmaceutical unit. For example, the tablets may be coated with shellac, sugar or both. In order to prevent the disintegration during transit through the upper gastrointestinal tract, the composition may be an enteric-coated formulation. A syrup or elixir may contain, in addition to the active ingredient, sucrose as a sweetening agent, methyl and propylparaben as preservatives, a colouring agent and a flavouring agent such as cherry or orange flavour.

In some embodiments, in the compositions of the present invention, the active ingredients are normally expressed in dosage units. The dosage unit may contain from 0.1 to 1000 mg of active ingredient or plant extract containing the active ingredient per dosage unit for daily administration.

In some embodiments of the composition of the invention, the dosage unit is a bag which can hold from 1 to 50 g, from 5 to 30 g of composition.

In some embodiments, the formulation contains quantities of active ingredients that will depend on the severity of COPD, the related symptoms, the condition, any drugs being also administered, individual health status and response to the association of active ingredients. In some embodiments, the dose is in the range from 0.001% by weight to about 60% by weight of the formulation.

According to some embodiments, the composition of the invention is a drug. According to some embodiments, the composition of the invention is a supplement, a nutritional product, a food for special medical purposes.

The following examples are provided primarily to illustrate the present invention and are not intended to limit the scope of protection as is clear from the appended claims.

The experimental model used by inventors is designed to demonstrate that by combining NAC and/or NAL and curcumin, a synergistic activity is obtained in preventing or treating the symptoms associated with COPD because each of these substances activates a cellular response to inflammation and facilitates the modulation of inflammatory genes. In greater detail, they act synergistically in inhibiting the activity of Nf-kb and in reducing the expression of inflammatory cytokines and the ability to induce the activity of Nrf2 and overexpress the intracellular levels of anti-inflammatory and antioxidant genes.

Following this action, the formulation of the present invention is effective in reducing the incidence of symptoms typical of COPD.

### EXAMPLE 1

Composition in granular form for the treatment of COPD and related symptoms having the following formulation

| | | |
|---|---|---|
| Whey proteins microfiltered at 92% | gr 11 | |
| N-Acetylcysteine | mg. 600 | |
| Turmeric titrated to 95% in Curcumin | mg. 523.6 | (equivalent to mg. 500 of Curcumin) |
| L-Carnitine | mg 500 | |
| Black pepper titrated to 95% in Piperine | mg. 5 | |
| Riboflavin (vit. B2) | mg. 1.2 | |

The daily dose is 2 bags containing the formulation described above.

### EXAMPLE 2

Food for special medical purposes in powder or granular form having the following formulation:

| | | |
|---|---|---|
| Whey proteins microfiltered at 92% | gr 11 | |
| N-Acetylcysteine | mg. 600 | |
| Turmeric titrated to 95% in Curcumin | mg. 523.6 | (equivalent to mg. 500 of Curcumin) |
| L-Carnitine | mg 300 | |
| Creatine | Mg 200 | |
| Plant extract from rosemary | Mg 150 | |
| Black pepper titrated to 95% in Piperine | mg. 5 | |
| Riboflavin (vit. B2) | mg. 1 | |

### EXAMPLE 3

### Experimental test

### Materials and methods

Curcumin (CUR), N-acetyl-L-cysteine (NAC) and glutathione in reduced (GSH) and oxidised (GSSG) form and other reagents were supplied by Sigma. Human alveolar epithelial cells A549 were purchased from American Type Culture Collection (ATCC CCL-185).

### Determination of intracellular glutathione

Levels of reduced (GSH) and oxidised (GSSG) Glutathione were measured after exposure of the cells for 6 hours to curcumin, NAC and curcumin and NAC association (ratio 1/1) using the method already described in Calabrese et al. 1998; Motterlini et al. 2000a).

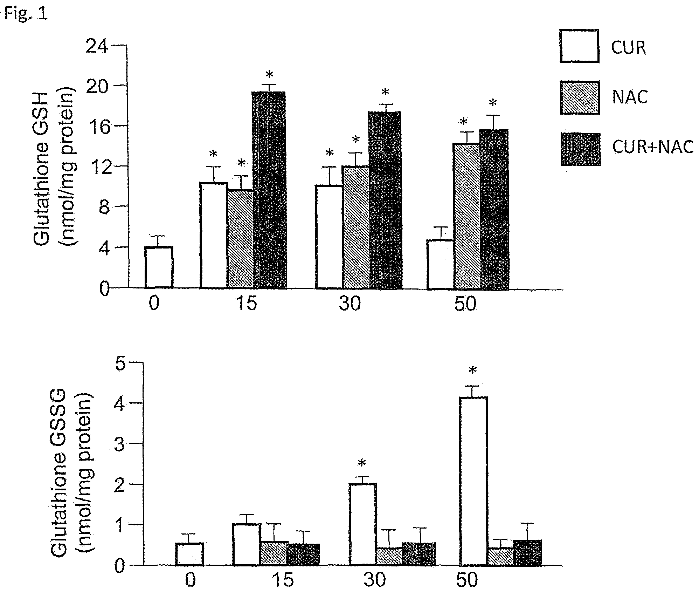

Cells harvested in cold PBS were frozen-thawed three times and an aliquot of this suspension was added to a buffer solution containing 12 ml of EDTA and 10 mM of 5,5-dithiobis- (2-nitrobenzoic acid). The total glutathione was measured by spectroscopy (optical density of 412 nm) using the glutathione reductase-recycling analysis. In order to determine the quantity of GSSG, an aliquot of the cell suspension was added to an equal volume of buffer containing EDTA and N-ethylmaleimide (10 mM). The sample was mixed and centrifuged and the supernatant was passed through a Sep-Pak C18 cartridge (Waters, Milford, MA) to remove the excess of N-ethylmaleimide. The sample was added to a container containing 5.5-dithiobis- (2-nitrobenzoic acid) and glutathione reductase and the analysis was carried out for the measurement of total glutathione. Intracellular glutathione was determined by comparison with a standard curve obtained with standard solutions of GSH and GSSG and was expressed as nmoles/mg protein. As can be seen in Fig. 1, GSH (A) and GSSG (B) levels were measured after exposure for 6 hours of the lung epithelial cells to different concentrations (0-50 µM) of Curcumin (CUR), NAC or CUR+NAC.

Each bar in Fig. 1 represents the average +/- of SEM of 4-5 experiments performed independently. *, p <0.05 versus 0 µM. The exposure to 15 and 30 µM of curcumin for 6 hours produced a significant increase in intracellular GSH and GSSG, while at 50 µM it caused oxidation without affecting the content of GSH. The exposure to NAC caused a similar effect on intracellular glutathione, causing an increase of GSH, and the effect was dose-dependent having a stronger effect at 50 µM. Furthermore, NAC did not increase the levels of GSSG in the same conditions. Surprisingly, the exposure to the combination of CUR+NAC showed at 15 and 3 µM a synergistic effect in the capacity of increasing GSH without inducing GSSG.

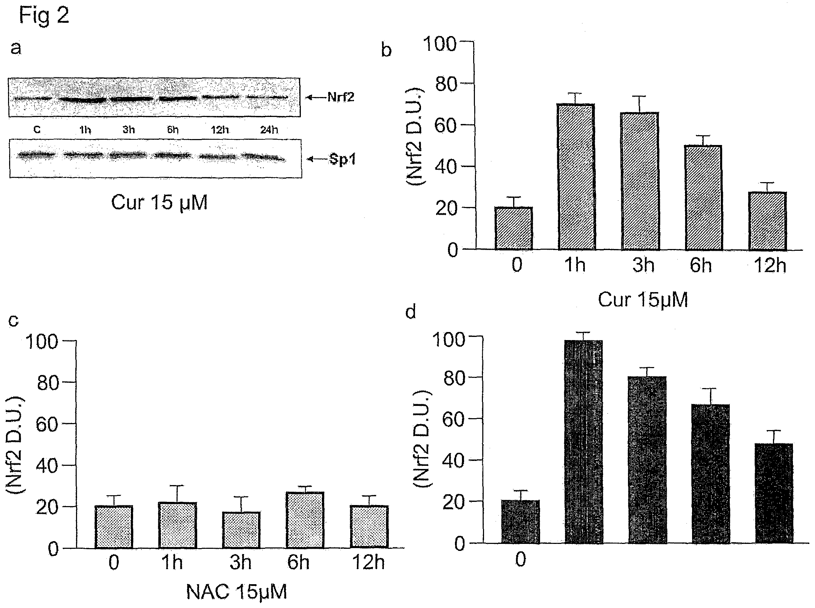

As shown in Fig. 2, the treatment with curcumin caused a significant time-dependent increase in the expression of the protein Nrf2 in the nucleic extracts. The quantification of three independent Western blots showed that after one hour of exposure to 15 µM of curcumin, the expression of Nrf2 increased significantly and it remained under-regulated up to 12h while the levels of the transcription factor Sp1 were stable. The NAC was not able to induce an increase of Nrf2 of the nucleus. The combination of NAC and CUR induced a slightly higher level of Nrf2 expression.

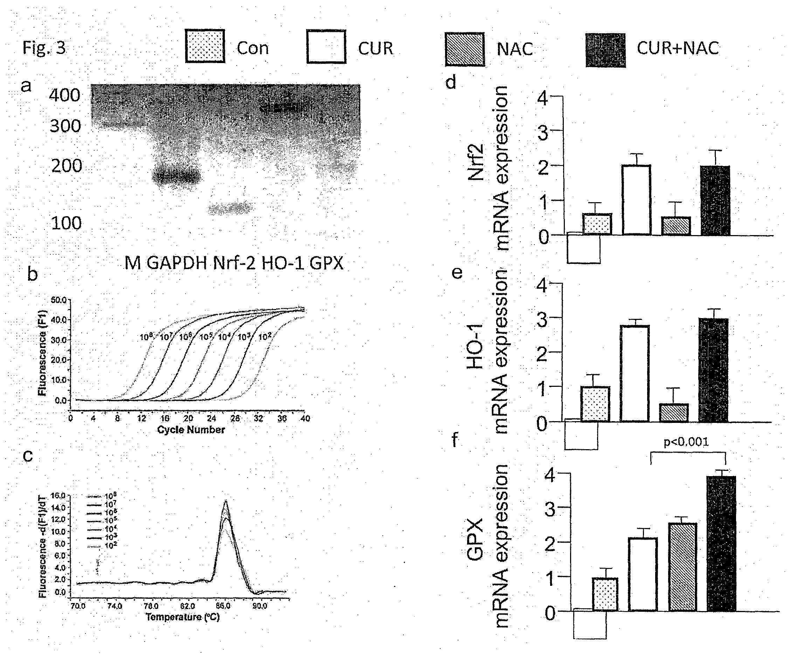

As can be seen in Fig. 3, the exposure of the cell line for 6 h at concentrations of 15 µM curcumin induced a significant increase (p<0.05) of Nrf2 HO-1 and glutathione reductase (GRX) mRNA, measured by quantitative real-time PCR and compared to the gene GAPDH. GRX is a key enzyme in the regulation of the cellular oxidative state, being responsible for the regeneration of GSH from GSSG and in the regeneration of dehydroascorbate. NAC, instead, is not active in inducing the expression of the gene Nrf2 and HO-1 at the same concentration but is able to induce GRX significantly. Surprisingly, the combination of CUR and NAC showed a synergistic effect in the capacity to increase GRX at a concentration of 15 µM.

## Claims

1. A nutritional composition comprising
a) a component with antioxidant activity and inhibiting protein catabolism comprising a synergistic combination of N-acetylcysteine or a salt thereof and curcumin, and
b) a protein-based component containing proteins obtained from whey for preventing or treating muscle hypotrophy, and vitamin B2 and carnitine and/or L-carnitine,
for use in the prevention and/or treatment of chronic obstructive pulmonary disease and correlated symptoms.

2. A composition for use according to claim 1, wherein said whey proteins include β lactoglobulin, α lactalbumin, serum albumin and mixtures thereof.

3. A composition for use according to claim 1 or 2, wherein component a) further comprises piperine.

4. A composition for use according to claim 3, wherein said piperine is contained in or derives from a plant extract of black pepper.

5. A composition for use according to any one of claims 1 to 4, wherein the salt of N-acetylcysteine is a lysine salt or Nacystelyn.

6. A composition for use according to any one of claims 1 to 5, wherein the curcumin is contained in a plant extract of Turmeric.

7. A composition for use according to any one of claims 1-6, further comprising a further active component selected from micronutrients, mineral salts, enzymes, vitamins and mixtures thereof.

8. A composition for use according to any one of claims 1 to 7, in granular form.

9. A composition for use according to claim 1 wherein said composition is a food for special medical purposes.

## Patentansprüche

1. Ernährungszusammensetzung aufweisend:
a) eine Komponente mit antioxidativer Aktivität und Proteinabbauhemmung aufweisend eine synergistische Kombination von N-Acetylcystein, oder einem Salz davon, und Kurkumin und
b) eine proteinbasierte Komponente, aufweisend aus Molke erhaltene Proteine zur Prävention oder Behandlung von Muskel-Hypotrophie, und Vitamin B2 und Carnitin und / oder L-Carnitin,
zur Verwendung bei der Prävention und / oder Behandlung chronisch obstruktiver Lungenerkrankung und korrelierten Symptomen.

2. Zusammensetzung zur Verwendung gemäß Anspruch 1, wobei die Molkeproteine β-Lactoglobulin, α-Lactalbumin, Serumalbumin und Mischungen davon beinhalten.

3. Zusammensetzung zur Verwendung gemäß Anspruch 1 oder 2, wobei die Komponente a) weiter Piperin aufweist.

4. Zusammensetzung zur Verwendung gemäß Anspruch 3, wobei das Piperin in einem Pflanzenextrakt von schwarzem Pfeffer enthalten ist oder davon stammt.

5. Zusammensetzung zur Verwendung gemäß einem der Ansprüche 1 bis 4, wobei das Salz des N-Acetylcysteins ein Lysinsalz oder Nacystelyn ist.

6. Zusammensetzung zur Verwendung gemäß einem der Ansprüche 1 bis 5, wobei das Kurkumin in einem Pflanzenextrakt von Kurkuma enthalten ist.

7. Zusammensetzung zur Verwendung gemäß einem der Ansprüche 1 bis 6, weiter aufweisend eine Wirkkomponente ausgewählt aus Spurenelementen, Mineralsalzen, Enzymen, Vitaminen und Mischungen davon.

8. Zusammensetzung zur Verwendung gemäß einem der Ansprüche 1 bis 7 in Granulatform.

9. Zusammensetzung zur Verwendung gemäß Anspruch 1, wobei die Zusammensetzung ein Nahrungsmittel für medizinische Zwecke ist.

## Revendications

1. Composition nutritionnelle comprenant
a) un composant avec une activité antioxydante et inhibant le catabolisme protéique comprenant une combinaison synergique de N-acétylcystéine ou d'un sel de celle-ci et de curcumine, et
b) un composant à base de protéines contenant des protéines obtenues de lactosérum pour la prévention ou le traitement de l'hypotrophie musculaire, et de la vitamine B2 et de la carnitine et/ou de la L-carnitine,
pour son utilisation dans la prévention et/ou le traitement de la bronchopneumopathie chronique obstructive et de symptômes associés.

2. Composition pour son utilisation selon la revendication 1, dans laquelle lesdites protéines de lactosérum comprennent la β-lactoglobuline, l'a-lactalbumine, la sérum albumine et des mélanges de celles-ci.

3. Composition pour son utilisation selon la revendication 1 ou 2, dans laquelle le composant a) comprend en outre de la pipérine.

4. Composition pour son utilisation selon la revendication 3, dans laquelle ladite pipérine est contenue dans ou provient d'un extrait végétal de poivre noir.

5. Composition pour son utilisation selon l'une quelconque des revendications 1 à 4, dans laquelle le sel de N-acétylcystéine est un sel de lysine ou Nacystélyn.

6. Composition pour son utilisation selon l'une quelconque des revendications 1 à 5, dans laquelle la curcumine est contenue dans un extrait végétal de curcuma.

7. Composition pour son utilisation selon l'une quelconque des revendications 1 à 6, comprenant en outre un composant actif supplémentaire sélectionné parmi des micronutriments, des sels minéraux, des enzymes, des vitamines et des mélanges de ceux-ci.

8. Composition pour son utilisation selon l'une quelconque des revendications 1 à 7, sous forme granulaire.

9. Composition pour son utilisation selon la revendication 1 dans laquelle ladite composition est un aliment destiné à des fins médicales spéciales.
